# EUROPEAN PATENT SPECIFICATION

(11) **EP 1 694 320 B1**
(45) Date of publication and mention of the grant of the patent: **09.07.2008**
(21) Application number: 04806247.5
(22) Date of filing: 17.12.2004
(51) Int. Cl.: A61K 31/191, A61P 37/08

(54) **USE OF COMPOSITIONS COMPRISING OLEANIC ACID AND URSOLIC ACID FOR THE PREPARATION OF A MEDICAMENT FOR THE TREATMENT OF HYPERSENSITIVITY AND HYPERREACTIVITY**
VERWENDUNG VON ZUSAMMENSETZUNGEN MIT OLEANSÄURE UND URSOLSÄURE ZUR HERSTELLUNG EINES MEDIKAMENTS ZUR BEHANDLUNG VON ÜBEREMPFINDLICHKEIT UND HYPERREAKTIVITÄT
UTILISATION DE COMPOSITIONS COMPRENANT DE L'ACIDE URSOLIQUE ET DE L'ACID OLEANIQUE POUR LA FABRICATION D'UN MEDICAMENT POUR LE TRAITEMENT DE L'HYPERSENSIBILITE ET DE L'HYPERREACTIVITE

(30) Priority: 19.12.2003 EP 03258087
(43) Date of publication of application: 30.08.2006
(73) Proprietor: Lipid Nutrition B.V., 1521 AZ Wormerveer (NL)
(72) Inventor: CAIN, Frederick W., c/o Loders Croklaan BV, NL-1521 AZ Womerveer (NL); STAM, Wiro, c/o Loders Croklaan BV, NL-1521 AZ Wormerveer (NL); SCHMID, Ulrike, c/o Loders Croklaan BV, NL-1521 AZ Wormerveer (NL); COLLINS, Geoff, Northants NN10 0TS (GB)
(74) Representative: Stevens, Ian Edward
(86) International application number: PCT/GB2004/005453
(87) International publication number: WO 2005/058302

(56) References cited:
- PATENT ABSTRACTS OF JAPAN vol. 1999, no. 04, 30 April 1999 (1999-04-30) & JP 11 012178 A (DAINIPPON PHARMACEUT CO LTD), 19 January 1999 (1999-01-19)
- RAPHAEL ET AL: "Effect of naturally occurring triterpenoids glycyrrhizic acid, ursolic acid, oleanolic acid and nomilin on the immune system" PHYTOMEDICINE, vol. 10, no. 6-7, 1 January 2003 (2003-01-01), pages 483-489, XP009032033 cited in the application
- DAI ET AL: "Effects of oleanolic acid on immune system and type I allergic reaction" ACTA PHARMACOLOGICA SINICA, vol. 10, no. 4, 1989, pages 381-384, XP009032032
- PATENT ABSTRACTS OF JAPAN vol. 0161, no. 14 (C-0921), 23 March 1992 (1992-03-23) & JP 03 287530 A (SNOW BRAND MILK PROD CO LTD), 18 December 1991 (1991-12-18) cited in the application
- DAI ET AL: "Inhibition of hypersensitivity reactions by oleanolic acid" ACTA PHARMACOLOGICA SINICA, vol. 9, no. 6, 1988, pages 562-565, XP009032031 cited in the application

## Description

This invention relates to the use of certain materials for the prevention or treatment of hypersensitivity and/or hyper-reactivity.

Hypersensitivity is a term used to describe an adaptive immune response that occurs in an exaggerated or inappropriate form and that can cause inflammatory reactions and tissue damage. Hypersensitivity is not usually manifested on first contact with a particular antigen but usually appears on subsequent contact. Hypersensitivity has been categorised into four types, Types I, II, III and IV. The first three types are antibody-mediated and the fourth is mediated primarily by T cells and macrophages. Type I hypersensitivity can occur when an IgE response is directed against environmental antigens such as pollen and house dust mites and can lead to an acute inflammatory reaction with symptoms such as asthma or rhinitis.

Hyper-reactivity is a characteristic of many inflammatory lung diseases and is an exaggerated degree of airway narrowing (Blease et al, Respir Res. 2000; 1 (1): 54-61). Hyper-reactivity can be defined as an exaggerated non-immune broncho-constrictive response to low concentrations of inhaled histamine or methacholine. Bronchial hyper-reactivity of this type is an almost constant manifestation of asthma (American Academy of Asthma and Allergy, Asthma and Immunology, In The News 2003, Mechanisms in bronchial hyper-reactivity).

Dai Y et al, Zhongguo Yao Li Xue Bao. 1988 Nov;9(6):562-5 describe the inhibition of hypersensitivity reactions by oleanolic acid. JP-A-3287530 (Snow Brand Milk Prod Co Ltd) discloses an immunosuppressive agent comprising ursolic acid or ketoursolic acid as an active ingredient.

Raphael et al, Phytomedicine, 10, 483-499, 2003, discloses the effect of glycyrrhizic acid, ursolic acid, oleanolic acid and nomilin on the immune system. The compounds are stated as increasing antibody production, whilst ursolic acid, oleanolic acid and nomilin are described as inhibiting a delayed type hypersensitivity reaction.

Other references describing health effects of ursolic acid and oleanolic acid include US 4752606, JP 09/040 689, JP 09/ 067 249, JP 09/020,674, CN 1 085 748, JP 1 039 973, JP 03/287 531, JP 03/287 43, EP 774255; JP 07/258 098, JP 07/048 260, JP 01/132 531, FR 2 535 203 and JP 1 207 262.

EP-A-1161879 describes a mixture comprising ursolic acid and oleanolic acid in a weight ratio of 1:99 to 99: 1, wherein the mixture contains less than 20 wt % of the natural apolar and/or low molecular weight components as present in natural extracts for ursolic acid and oleanolic acid.

EP-A-1123659 describes blends of fats with a composition comprising ursolic acid and oleanolic acid. The compositions display high crystallisation rates.

JP-A-11012178 discloses medical agents comprising an oleanolic derivative (3-O-monodesmoside).

Dai Y *et al*, 1989; 10(4): 381-4 describes the inhibition of hypersensitivity reactions by oleanolic acid.

It has been found that compositions comprising ursolic acid and oleanolic acid, together with other triterpenoic acids (sometimes also referred to as triterpenic acids), which can be obtained as extracts from natural materials, can be used in the treatment and/or prevention of hypersensitivity and/or hyper-reactivity.

According to the invention in a first aspect there is provided the use of a material comprising from 30 to 80 % by weight of ursolic acid, from 2 to 25 % by weight of oleanolic acid and from 1 to 68% by weight of triterpenoic acids other than ursolic acid or oleanolic acid, or salts or esters of any of these acids, said percentages being based on total weight of said acids or salts or esters and the percentages of said acids or salts or esters adding up to 100%, in the manufacture of a composition for the prevention or treatment of hypersensitivity and/or hyper-reactivity. The invention also contemplates this material for use in the prevention or treatment of hypersensitivity and/or hyper-reactivity.

The hypersensitivity is typically an allergy. It is preferred that the material has the effect of decreasing the level of IgE when taken by a subject (preferably a mammal, more preferably a human). It is believed that this reduction in IgE levels is at least partly responsible for the reduction in hypersensitivity.

A preferred effect for the material of the invention is in inhibiting or preventing constriction of the bronchial tubes, for example in the treatment of asthma. The material may reduce airway hyperresponsiveness to inhaled irritants and viruses as well as changes in temperature.

The invention may be useful in the treatment and/or prevention of hypersensitivity and/or hyper-reactivity. Symptoms that may be prevented and/or treated according to the invention include asthma, coughing (including dry coughs, tickly coughs and more productive coughs), wheezing and runny nose.

Oral administration or consumption of the material and/or composition are preferred. The material and/or composition are therefore preferably adapted for oral administration.

The material may be formulated in a number of different product forms" including for example a pharmaceutical composition, a foodstuff or a food supplement. Preferably, the pharmaceutical composition, foodstuff or food supplement comprises a total amount of ursolic and oleanolic acid in a dosage amount of from 0.02 g to 20 g per day. The compositions may comprise one or more farther components that are useful in the treatment and/or prevention of hypersensitivity or hyper-reactivity.

Compositions of the invention may also comprise one or more further components selected from conjugated linoleic acid (CLA), fish oil, conjugated trienoic acids and mixtures thereof.

A preferred composition according to the invention is a foodstuff. Foodstuffs include liquids (e.g, beverages) and solids. Suitably, foodstuffs will be packaged and labelled as foodstuffs. Conventional foodstuffs may incorporate the material of the invention in a suitable amount.

Pharmaceutical compositions may, for example, be in the form of tablets, pills, capsules, caplets, multiparticulates including: granules, beads, pellets and micro-encapsulated particles; powders, elixirs, syrups, suspensions, emulsions and solutions. Preferred product forms are tablets, capsules, solutions and emulsions. Pharmaceutical compositions will comprise a pharmaceutically acceptable diluent or carrier. Pharmaceutical compositions are preferably adapted for administration parenterally (e.g., orally). Orally administrable compositions may be in solid or liquid form and may take the form of tablets, powders, suspensions and syrups. Optionally, the compositions comprise one or more flavouring and/or colouring agents. Pharmaceutically acceptable carriers suitable for use in such compositions are well known in the art of pharmacy. The pharmaceutical compositions of the invention may contain 0.1-99% by weight of the material of the invention. Pharmaceutical compositions of the invention are generally prepared in unit dosage form.

Further examples of compositions of the invention are food supplements, such as in the form of a soft gel or a hard capsule comprising an encapsulating material selected from the group consisting of gelatin, starch, modified starch, starch derivatives such as glucose, sucrose, lactose and fructose. The encapsulating material may optionally contain cross-linking or polymerizing agents, stabilizers, antioxidants, light absorbing agents for protecting light-sensitive fills, preservatives and the like. Preferably, the unit dosage of the composition of the invention in the food supplements is from 1mg to 1000mg (more preferably from 100mg to 750mg).

Preferred foodstuffs include those selected from the group consisting of margarines, fat continuous or water continuous or bicontinuous spreads, fat reduced spreads, confectionery products such as chocolate or chocolate coatings or chocolate filling or bakery fillings, ice creams, ice cream coatings, ice cream inclusions, dressings, mayonnaises, cheeses, cream alternatives, dry soups, drinks, cereal bars, sauces, snack bars, dairy products, clinical nutrition products and infant formulations. Foodstuffs preferably comprise from 0.001 % to 5 % by weight (more preferably 0.01 % to 4 %, even more preferably 0.1 % to 3 %, most preferably 0.1 % to 2 % or 0.1 % to 1 % by weight) of the material of the invention.

Certain preferred food products for use in the invention comprise the material in the form of a blend with other components in particular as a blend with glycerides, preferably triglycerides. The blend preferably contains 1 to 99 wt %, more preferably 5 to 80 wt % of one or more components selected from mono-, di-, and triglycerides. The glyceride part of this blend preferably displays a solid fat content measured by NMR-pulse on a non-stabilised fat at the temperature indicated of 5 to 90 at 5°C,2 to 80 at 20°C and less than 15, preferably less than 10 at 35°C.

The solid fat content is measured by the well known NMR-pulse technique on a fat that is not stabilised, this means that the measurement was performed on a fat that was subjected to the following treatment: melt at 80°C, keep it at 80°C for 15 minutes, cool it to 0°C and keep it at 0°C for 30 minutes, heat it to measurement temperature and keep it at tills temperature for 30 minutes and measure the N-value at this temperature.

Preferred blends are blends comprising components A, B and C, wherein:
A is the material according to the invention
B is a solid fat with an N20 of more than 20, preferably more than 45, most preferably more than 60 and
C is a fat having at least 40 wt % of fatty acids with 18 C-atoms and having one to three double bonds.

A is typically present in amounts of more than 0.1 wt %, preferably 0.1 to 20 wt %, most preferably 0.2 to 10 wt %. B may be present in amounts of 8 to 90 wt %, preferably 25 to 75 wt %, most preferably 40 to 70 wt %. C may be present in amounts of 0 to 85 wt %, preferably 15 to 65 wt %, most preferably 20 to 50 wt %.

In these blends, the fat component B is preferably selected from the group consisting of palm oil; palm oil fractions; cocoa butter equivalents; palm kernel oil; fractions of palm kernel oil; hardened vegetable oils such as hardened palm oil; hardened fractions of palm oil; hardened soybean oil; hardened sunflower oil; hardened rape seed oil; hardened fractions of soybean oil; hardened fractions of rapeseed oil; hardened fractions of sunflower oil; mixtures of one or more of these oils and interesterified mixtures thereof.

Fat component C in general will be a liquid oil and is preferably selected from the group consisting of sunflower oil; olive oil; soybean oil; rape seed oil; palm oil olein; cotton seed oil; olein fractions from vegetable oils; high oleic vegetable oils such as HOSF (=high oleic sunflower oil) or HORP (=high oleic rape seed oil); fish oils; fish oil concentrates and conjugated linoleic acid (CLA) -glycerides.

The blends comprising components A, B and C as disclosed above have excellent properties for application in food products containing a fat phase.

The blends can also contain other known additives such as preservatives, colouring agents, stabilisers, vitamins and minerals.

The material of the invention comprises ursolic acid, oleanolic acid and other triterpenoic acids, or esters or salts of any of these acids and it is believed that the mixture of acids provides advantages over the corresponding acids used alone. The material of the invention comprises 30 % to 80 % by weight of ursolic acid, from 2 % to 25 % by weight of oleanolic acid and from 1 % to 68% by weight of triterpenoic acids other than ursolic acid or oleanolic acid, or salts or esters of any of these acids. Triterpenoic acids other than ursolic acid and oleanolic acid include maslinic acid. Preferably, the material of the invention comprises 40 to 80 % (such as 40 to 70 %) by weight of ursolic acid, from 5 to 15 % by weight of oleanolic acid and from 10 to 50% (such as 25 to 50 %) by weight of triterpenoic acids other than ursolic acid or oleanolic acid, or salts or esters of any of these acids. More preferably, the material of the invention comprises 45 to 65 % by weight of ursolic acid, from 5 to 15 % by weight of oleanolic acid and from 15 to 50% by weight of triterpenoic acids other than ursolic acid or oleanolic acid, or salts or esters of any of these acids. Even more preferably, the material of the invention comprises 50 to 60 % by weight of ursolic acid, from 8 to 12 % by weight of oleanolic acid and from 30 to 40% by weight of triterpenoic acids other than ursolic acid or oleanolic acid, or salts or esters of any of these acids. These percentages are based on the total weight of these acids in the material. Based on the total weight of the material, the material preferably comprises ursolic acid, oleanolic acid and other triterpenoic acids (or salts or esters thereof) in a total amount of 30% to 100% by weight, more preferably 30% to 95% by weight, even more preferably 30% to 90% by weight, such as 30% to 70% by weight or 40% to 60% by weight. Other components of the material may include triglycerides, polar materials and minor components.

Derivatives of ursolic acid, oleanolic acid and other triterpenoic acids that are suitable for use in the invention include salts derived from the acids which are non-toxic at the levels used and do not inhibit the activity of the acids. Suitable derivatives include esters (e.g., with an alcohol comprising from 1 to 22 carbon atoms, such as C₁ to C₆ alkyl esters) and salts (e.g., sodium or potassium salts). Preferably, however, the material comprises the acids in the form of the free acid. Sodium salts of ursolic acid, oleanolic acid and the other triterpenoic acids, optionally in admixture with the free acids, are also preferred.

The material of the invention is preferably extractable or otherwise obtainable from fruit skin and preferably has substantially the same ratio of ursolic acid to oleanolic acid as is present in the natural fruit skin. The fruit skin is suitably derived from fruit selected from apples, cranberries, olives, grapes and mixtures thereof. The material may have been treated as described in EP-A-1161879 in order to improve taste properties. The triterpenoic acids in the material of the invention are therefore preferably obtainable from fruit skin.

The material of the invention is preferably obtained by a process comprising treating fruit skin (optionally dried and optionally washed with water and/or a solvent which is immiscible with water in equimolar amounts at 25 °C, such as hexane) with an organic solvent (preferably acetone or a mixture of acetone and water), removing the solvent to form an extract, optionally drying and optionally further purifying the dried extract. Further purification steps include, for example: dissolving the dried extract in acetone and filtering the resulting solution, after which the solvent is removed; and washing the dried extract with water (e.g., at an elevated temperature such as 40 to 90°C).

Derivatives of the acids can be prepared correspondingly. For example, a material comprising the sodium salt of ursolic acid, oleanolic acid and other triterpenoic acids can be prepared by treating fruit skin (optionally dried and optionally washed with water and/or a solvent which is immiscible with water in equimolar amounts at 25 °C, such as hexane) with an organic solvent (preferably acetone or a mixture of acetone and water) in the presence of a basic sodium salt (e.g., sodium hydroxide) at a suitable concentration (such as 0.1 to 1 M, preferably 0.4 to 0.6 M). Any solids may be removed from the resulting mixture (e.g., by filtration), and the solvent removed (e.g., under vacuum, preferably at about 60 °C) to form a slurry. The resulting slurry may be filtered again and, optionally, washed with water to form the sodium salt mixture as the residue which may be dried and optionally further purified.

The following non-limiting examples illustrate the invention. In the examples and throughout this specification, all percentages, parts and ratios are by weight unless indicated otherwise.

### Examples

The examples refer to the accompanying drawings in which:
Figure 1 is a plot of barometric whole-body plethysmography and increases in enhanced pause against concentration of metacholine for groups before and after treatment according to the invention.
Figure 2 is a bar chart showing IgE serum levels before and after challenge with ovalbumin (OVA) in four different groups, one control and three according to the invention.
Figure 3 is a bar chart showing the effect of a material according to the invention, which is in the form of a sodium salt of an apple extract, in reducing the bronchial hyperreactivity to metacholine compared to a control.

### Example 1

Approximately 25000kg of apple pomace (predominantly peel and core) was oven dried at 100-150 °C to produce approximately 6000kg of dried pomace containing less than 10wt% moisture. The dried pomace was ground to pass through a 20mm screen leaving the majority of pips intact.

Extraction of the dried/ground pomace was performed in a ten step counter current extractor with hot acetone at 50- 55 °C until more than 95% of acetone extractables were removed. Acetone was removed in a pair of thin layer evaporators under vacuum. At the same time, purified water was added to make a watery suspension of non-polar compounds including triterpenoids.

The resulting water suspension containing 5 to 15% solids was allowed to cool then centrifuged to remove the majority of water present resulting in a wet cake. Additional hot purified water was flushed through the cake to remove the majority of remaining polar compounds including sugars.

The wet cake was suspended in purified water to enable pumping then spray dried to produce a fine dry powder (less than 0.5% moisture). Fine dust from the filter bags was recombined with the rest of the product. This resulted in 617kg of dry apple extract.

For further purification, 120 kg of the extract was dissolved in 9600 kg acetone at 40°C. Then, 7.2 kg Norit SA4 active carbon (9kg) was added and the mixture was stirred for a period of 6 hours at 47 to 53 °C. The active carbon was removed with the aid of Arbocel 00 filter aid in combination with two in series 1-3 µm filter plates.

Acetone was removed in a pair of thin layer evaporators under vacuum. At the same time, purified water was added to make a watery suspension of non-polar compounds including triterpenoids.

This was then spray dried to produce a fine dry powder (<0.5% moisture). Fine dust from the filter bags was recombined with the rest of the product. In total, 67.2 kg of purified extract was obtained.

The resulting product contained about 55% by weight ursolic acid, about 10% by weight oleanolic acid and about 35% by weight of other triterpenoic acids, based on the total weight of these acids in the material. Triterpenoic acids formed about 49% by weight of the product based on the total weight of the material.

### Example 2

Patients suffering from allergic asthma are characterized by the presence of allergen specific IgE antibodies and the presence of non-specific airway hyper-reactivity. Two mice models were used to investigate the effect of the apple pomace extract containing ursolic acid on IgE levels (Example 3) and on the development of airway hyper responsiveness to metacholine (Example 2).

BALB/cByJIco mice were obtained. Upon arrival the animals were weighed and marked. Animals were put on diets containing the active ingredient at 7-4 days before start of the protocol.

### Protocol 1: Effect on airway hyper-reactivity

Animals (n=14 per group) were fed a diet mixed with either:
*Group 1*: no additive
*Group* 2: Sodium salt of ursolic and oleanolic acid at a dose of 0.75%
*Group 3:* 2.5% extract of Example 1
*Group 4:* 4% extract of Example 1

Day 0 - 14: Seven times on alternating days one intraperitoneal injection with 10 µg ovalbumin (OVA).
Day 27: Collect blood for determination of OVA-specific immunoglobulin.
Day 31: Assessment of basal airway reactivity to metacholine.
Day 38 - 45: Eight days on alternating days challenge with 2 mg OVA/ ml saline by inhalation.
Day 46: Assessment of basal airway reactivity to metacholine

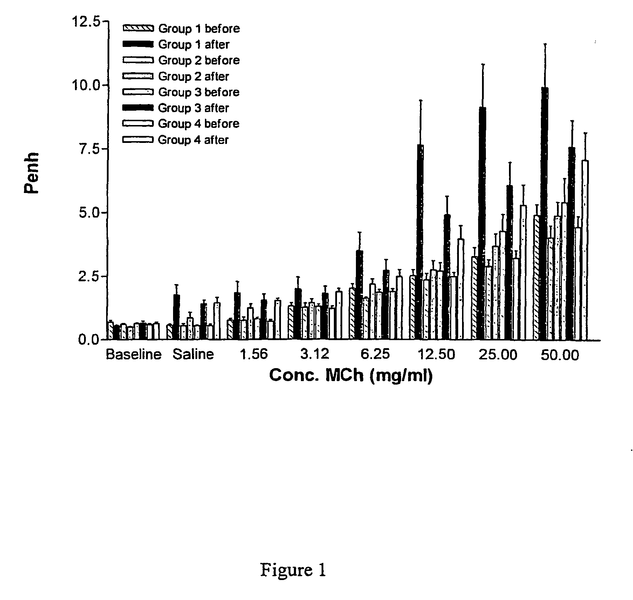

The results are shown in Figure 1.

Using barometric whole-body plethysmography and increases in enhanced pause (Penh) as an index of airway obstruction, airway reactivity was assessed. From the results in Figure 1 it is clear that the control mice (group 1) develop airway hyper responsiveness to increasing doses of metacholine. Furthermore, it is quite clear that treatment with pure ursolic acid salt (group 2) and the 2.5% and 4% extract treatment (groups 3 and 4, respectively) strongly inhibited the development of airway hyper-reactivity.

This example shows that the extract according to the invention reduced airway hyper-reactivity in a mouse model of asthma.

### Example 3

The effect of the extract on allergen specific IgE levels was studied.
6 week old SPF-male BALB/c mice were used. Four groups were used in the study:
i) control
ii) extract 0.5% UA of the diet
iii) extract 1% UA of the diet
iv) extract 2.5% UA of the diet

The following protocol was employed:
day 0 to 7: i.p. sensitisation with OVA (allergen) + alum
day 20: measure IgE before challenge
day 21;24,27: challenge with OVA-aerosol or saline 3 times on 3 days
day 28: measure IgE post-challenge

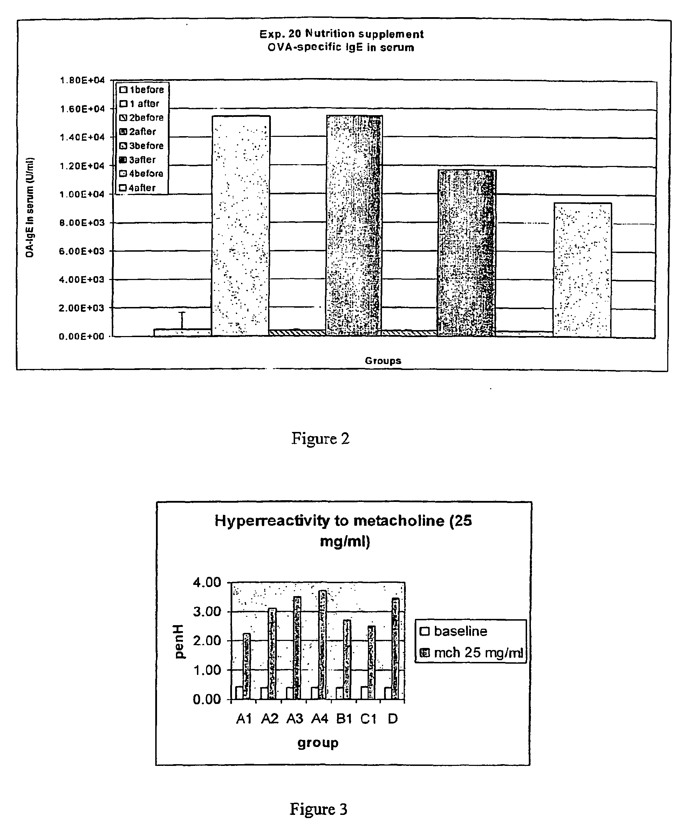

The results are shown in Figure 2.

From Figure 2, it is clear that after challenge with OVA the specific IgE is induced in all groups (difference in before and after). However, it is also clear that incorporation of ursolic acid through the diet resulted in a dose-dependent decrease in IgE levels.

This example shows that the extract according to the invention reduced IgE levels in a mouse model.

### Example 4

The following is an example of a filled gelatin capsule according to the invention. An extract produced according to Example 1 is encapsulated into a gelatin capsule according to methods well-known in the art. The resulting encapsulated product contains 500 mg of the mixture of the extract and one tablet can be taken up to four times daily by an adult human.

### Example 5

The following is an example of a margarine-type spread according to the invention. The spread can be prepared according to the procedure described in Example 14 of WO 97/18320.

| Fat Phase: | |
|---|---|
| Fat Blend* | 40 % |
| Hymono 7804 (emulsifier) | 0.3 % |
| Colour (2% β-carotene) | 0.02% |
| Total | 40.32 % |

| | |
|---|---|
| *87:13 by weight sunflower oil and hardstock | |

| Aqueous Phase (to pH 5.1): | |
|---|---|
| Water | 55.94 % |
| Extract of Example 1 | 0.5 % |
| Skimmed Milk Powder | 1.5 % |
| Gelatin (270 bloom) | 1.5 % |
| Potassium Sorbate | 0.15 % |
| Citric Acid Powder | 0.07 % |
| Total | 59.66 % |

### Example 6

### Preparation of sodium salt of triterpenic acids

40 g of hexane washed apple extract was fully solubilised in 400ml of acetone at room temperature. 290 ml of 0.5M NaOH was added in approximately 10 minutes and the mixture was stirred for approximately 20 minutes. The reaction solution was filtered over a filter paper and the acetone was removed at 60°C under vacuum. The resulting slurry was filtered over a Buchner filter and the filter cake washed with distilled cold water and dried at room temperature.

### Example 7

### Animal efficacy study

Patients suffering from allergic asthma are characterized by the presence of allergen specific IgE antibodies, hyperreactivity of the airways and the presence of a chronic inflammation in the airways with a predominance of eosinophils. A well characterized mouse model (BALB/c) has been developed with similar characteristics as asthmatic patients.

Mice were sensitized to ovalbumine (OVA) without any adjuvant by 7 intraperitoneal injections on alternate days from day 1 until day 13. Subsequently, these sensitized mice were challenged by exposure to an aerosol containing OVA from day 33 until day 40. Mice were sacrificed on day 41. Bronchial hyperreactivity for metacholine was assessed on day 41.

The current study consisted of 7 treatment groups of 14 animals per group. Extracts were mixed through the diet and were fed from day 0 until the end of the study.

The following groups were tested:
Group A1: Sodium salt of apple extract, wherein 0.85% of triterpenic acids are in the diet
Group A2: Sodium salt of apple extract, wherein 0.24% of triterpenic acids are in the diet
Group A3: Sodium salt of apple extract, wherein 0.085% of triterpenic acids are in the diet
Group A4: Sodium salt of apple extract, wherein 0.05% of triterpenic acids are in the diet
Group B1: Commercial available sodium salt (Selco) comprising ursolic acid and oleanolic, wherein 0.85% of these triterpenic acids are in the diet
Group C1: apple extract, wherein 0.85% of triterpenic acids are in the diet
Group D: control

The composition for groups A1 to A4 was produced according to Example 6.

The following table shows the levels of triterpenoic acids in the composition used.

Levels of triterpenes in the preparations (%)

| | Ursolic acid in wt% | Oleauolic acid in wt% | Other triterpenic acids in wt% | Total amount of triterpenic acids in wt% |
|---|---|---|---|---|
| Sodium salt of apple extract (A1 -A4) | 65.8 | 11.2 | 14.5 | 91.5 |
| Apple extract (C1) | 27.5 | 5.8 | 21.4 | 54.7 |
| Commercially available sodium salt of ursolic and oleanolic acid (B1) | 63.2 | 21.7 | 0.7 | 85.6 |

Figure 3 shows the results of this experiment. Figure 3 demonstrates that the sodium salt of apple extract (group A1) reduces the bronchial hyperreactivity to metacholine compared to the control (group D). Extract A1 is most potent, followed by the apple extract (C1), followed by the commercially available salt material (B1). For groups A1 to A4 there is a clear dose response relationship. It can be concluded that both the apple extracts, salt and non-salt, at a dose of 0.85% in the diet containing a combination of triterpenic acids are more active than the commercially available sodium salt comprising mainly ursolic and oleanolic acid. (The Penh value is a measure for airway reactivity and metacholine is a bronchoconstrictor agent.) The data show that the compositions of the invention, as exemplified by group A1, are superior to closely related compositions having different levels of ursolic and oleanolic acids and/or lower levels of other triterpenoic acids.

## Claims

1. Use of a material comprising from 30 to 80 % by weight of ursolic acid, from 2 to 25 % by weight of oleanolic acid and from 1 to 68% by weight of triterpenoic acids other than ursolic acid or oleanolic acid, or salts or esters of any of these acids, said percentages being based on total weight of said acids or salts or esters and the percentages of said acids or salts or esters adding up to 100%, in the manufacture of a composition for the prevention or treatment of hypersensitivity and/or hyper-reactivity.

2. Use as claimed in claim 1, wherein the hypersensitivity is an allergy.

3. Use as claimed in claim 1 or claim 2, wherein the composition decreases the level of IgE.

4. Use as claimed in any one of claims 1 to 3, wherein the composition inhibits or prevents constriction of the bronchial tubes.

5. Use as claimed in any one of claims 1 to 4, wherein the composition is a pharmaceutical composition, a foodstuff or a food supplement.

6. Use as claimed in any one of claims 1 to 5, wherein ursolic acid and/or oleanolic acid are in the form of the sodium or potassium salt.

7. Use as claimed in any one of claims 1 to 6, wherein ursolic acid and/or oleanolic acid are in the form of an ester with an alcohol comprising from 2 to 22 carbon atoms.

8. Use as claimed in any one of claims 1 to 7, wherein the composition comprises a total amount of ursolic and oleanolic acid in a dosage amount of from 0.02 g to 20 g per day.

9. Use as claimed in any one of claims 1 to 8, wherein the ursolic acid and oleanolic acid are extractable from fruit skin.

10. Use as claimed in claim 9, wherein the fruit skin is derived from fruit selected from apples, cranberries, olives, grapes and mixtures thereof

11. Use as claimed in any one of claims 1 to 10, wherein the composition is a foodstuff selected from the group consisting of margarines, fat continuous or water continuous or bicontinuous spreads, fat reduced spreads, confectionery products such as chocolate or chocolate coatings or chocolate filling or bakery fillings, ice creams, ice cream coatings, ice cream inclusions, dressings, mayonnaises, cheeses, cream alternatives, dry soups, drinks, cereal bars, sauces, snack bars, dairy products, clinical nutrition products and infant formulations.

12. Use as claimed in any one of claims 1 to 11, wherein the composition is a pharmaceutical composition in the form of a tablet, capsule, solution or emulsion.

13. Use as claimed in any one of claims 1 to 12, wherein the composition is a food supplement in the form of a soft gel or a hard capsule comprising an encapsulating material selected from the group consisting of gelatin, starch, modified starch, starch derivatives such as glucose, sucrose, lactose and fructose.

14. Use as claimed in any one of claims 1 to 13, wherein the composition comprises one or more further components selected from conjugated linoleic acid (CLA), fish oil, conjugated trienoic acids and mixtures thereof.

## Patentansprüche

1. Verwendung eines Materials, das von 30 bis 80 Gewichts-% Ursolsäure, von 20 bis 25 Gewichts-% Oleansäure und von 1 bis 68 Gewichts-% andere Triterpensäuren als Ursolsäure oder Oleansäure oder Salze oder Ester von jeder dieser Säuren enthält, wobei die Prozentsätze auf dem Gesamtgewicht der Säuren oder Salze oder Ester basieren und die Prozentsätze der Säuren oder Salze oder Ester zusammen 100 % ergeben, bei der Herstellung einer Zusammensetzung für die Verhinderung oder Behandlung von Überempfindfichkeit und/oder Hyperreagibilität.

2. Verwendung wie in Anspruch 1 beansprucht, wobei die Überempfindlichkeit eine Allergie ist.

3. Verwendung wie in Anspruch 1 oder 2 beansprucht, wobei die Zusammensetzung das Niveau an IgE senkt.

4. Verwendung wie in einem der Ansprüche 1 bis 3 beansprucht, wobei die Zusammensetzung die Verengung der Bronchien inhibiert oder verhindert.

5. Verwendung wie in einem der Ansprüche 1 bis 4 beansprucht, wobei die Zusammensetzung eine pharmazeutische Zusammensetzung, ein Nahrungsmittel oder ein Nahrungsergänzungsmittel ist.

6. Verwendung wie in einem der Ansprüche 1 bis 5 beansprucht, wobei Ursolsäure und/oder Oleansäure in der Form des Natrium- oder Kaliumsalzes vorliegt.

7. Verwendung wie in einem der Ansprüche 1 bis 6 beansprucht, wobei Ursolsäure und/oder Oleansäure in der Form eines Esters mit einem Alkohol vorliegt, der von 2 bis 22 Kohlenstoffatome aufweist.

8. Verwendung wie in einem der Ansprüche 1 bis 7 beansprucht, wobei die Zusammensetzung eine Gesamtmenge an Ursol- und Oleansäure in einer Dosierungsmenge von ungefähr 0,02 g bis 20 g pro Tag aufweist.

9. Verwendung wie in einem der Ansprüche 1 bis 8 beansprucht, wobei die Ursolsäure und die Oleansäure von Fruchthäuten extrahierbar sind.

10. Verwendung wie in Anspruch 9 beansprucht, wobei die Fruchthäute von Früchten erhalten sind, die aus Äpfeln, Kranbeeren, Oliven, Trauben und Mischungen davon ausgewählt sind.

11. Verwendung wie in einem der Ansprüche 1 bis 10 beansprucht, wobei die Zusammensetzung ein Nahrungsmittel ist, welches aus der Gruppe ausgewählt ist, die besteht aus Margarinen, fettkontinuierlichen, wasserkontinuierlichen oder bikontinuierlichen Aufstrichen, fettreduzierten Aufstrichen, Süßwaren, so wie Schokolade oder Schokoladebeschichtungen oder Schokoladefüllungen oder Backfüllungen, Eiscremes, Eiscremebeschichtungen, Eiscremeeinschlüssen, Dressings, Majonäsen, Käsen, Sahnealternativen, Trockensuppen, Getränken, Müsliriegeln, Saucen, Zwischenmahlzeitriegeln, Molkereiprodukten, klinischen Nahrungsmitteln und Kinderformulierungen.

12. Verwendung wie in einem der Ansprüche 1 bis 11 beansprucht, wobei die Zusammensetzung eines pharmazeutische Zusammensetzung in der Form einer Tablette, Kapsel, Lösung oder Emotion ist.

13. Verwendung wie in einem der Ansprüche 1 bis 12 beansprucht, wobei die Zusammensetzung ein Nahrungsergänzungsmittel in Form einer Weichgel- oder Hartkapsel ist, die ein einkapselndes Material aufweist, welches aus der Gruppe ausgewählt ist, die besteht aus Gelatine, Stärke, modifizierter Stärke und Stärkederivaten, so wie Glukose, Saccharose, Lactose und Fructose.

14. Verwendung wie in einem der Ansprüche 1 bis 13 beansprucht, wobei die Zusammensetzung eine oder mehrere weitere Komponenten aufweist, die aus konjugierter Linolsäure (CLA), Fischöl, konjuigierten Triensäuren und Mischungen davon ausgewählt ist.

## Revendications

1. Utilisation d'un matériau comprenant de 30 à 80% en poids d'acide ursolique, de 2 à 25% en poids d'acide oléanolique et de 1 à 68% en poids d'acides triterpénoïques autres que l'acide ursolique ou l'acide oléanolique, ou des sels ou esters de l'un quelconque de ces acides, lesdits pourcentages étant sur la base du poids total desdits acides ou sels ou esters, et les pourcentages desdits acides ou sels ou esters se montant à 100%, dans la fabrication d'une composition pour la prévention ou le traitement d'une hypersensibilité et/ou d'une hyperréactivité.

2. Utilisation selon la revendication 1, dans laquelle l'hypersensibilité est une allergie.

3. Utilisation selon la revendication 1 ou la revendication 2, dans laquelle la composition diminue le taux d'IgE.

4. Utilisation selon l'une quelconque des revendications 1 à 3, dans laquelle la composition inhibe ou prévient la constriction des tubes bronchiques.

5. Utilisation selon l'une quelconque des revendications 1 à 4, dans laquelle la composition est une composition pharmaceutique, une denrée alimentaire ou un complément alimentaire.

6. Utilisation selon l'une quelconque des revendications 1 à 5, dans laquelle l'acide ursolique et/ou l'acide oléanolique sont sous la forme du sel de sodium ou de potassium.

7. Utilisation selon l'une quelconque des revendications 1 à 6, dans laquelle l'acide ursolique et/ou l'acide oléanolique sont sous la forme d'un ester avec un alcool comprenant de 2 à 22 atomes de carbone.

8. Utilisation selon l'une quelconque des revendications 1 à 7, dans laquelle la composition comprend une quantité totale d'acides ursolique et oléanolique en une quantité posologique de 0,02 g à 20 g par jour.

9. Utilisation selon l'une quelconque des revendications 1 à 8, dans laquelle l'acide ursolique et l'acide oléanolique sont susceptibles d'être extraits de la peau de fruit.

10. Utilisation selon la revendication 9, dans laquelle la peau de fruit est issue de fruit choisi parmi les pommes, les airelles rouges, les olives, le raisin et les mélanges de ceux-ci.

11. Utilisation selon l'une quelconque des revendications 1 à 10, dans laquelle la composition est une denrée alimentaire choisie dans le groupe constitué par les margarines, les produits à tartiner à matière grasse continue ou eau continue ou bicontinue, les produits à tartiner à matière grasse réduite, les produits de confiserie tels que le chocolat ou les enrobages de chocolat ou les fourrages au chocolat ou les fourrages de pâtisserie, les crèmes glacées, les enrobages de crème glacée, les inclusions de crème glacée, les vinaigrettes, les mayonnaises, les fromages, les succédanés de la crème, les soupes en poudre, les boissons, les barres céréalières, les sauces, les snacks, les produits laitiers, les produits de nutrition clinique et les formulations pour nourrissons.

12. Utilisation selon l'une quelconque des revendications 1 à 11, dans laquelle la composition est une composition pharmaceutique sous la forme d'un comprimé, d'une capsule, d'une solution ou d'une émulsion.

13. Utilisation selon l'une quelconque des revendications 1 à 12, dans laquelle la composition est un complément alimentaire sous la forme d'un gel mou ou d'une capsule dure comprenant un matériau d'encapsulation choisi dans le groupe constitué par la gélatine, l'amidon, un amidon modifié, les dérivés de l'amidon tels que le glucose, le saccharose, le lactose et le fructose.

14. Utilisation selon l'une quelconque des revendications 1 à 13, dans laquelle la composition comprend un ou plusieurs autres constituants choisis parmi l'acide linoléique conjugué (CLA), une huile de poisson, les acides triénoïques conjugués et les mélanges de ceux-ci.
